## Europäisches Patentamt
## European Patent Office
## Office européen des brevets

(19)

(11) Veröffentlichungsnummer: **0 221 592**
**B1**

(12)

# EUROPÄISCHE PATENTSCHRIFT

(45) Veröffentlichungstag der Patentschrift:
09.08.89

(51) Int. Cl.⁴: **A 61 B  8/08, A 61 B 17/22**

(21) Anmeldenummer: 86201736.5

(22) Anmeldetag: 08.10.86

(54) Verfahren zur Ultraschallkontrolle bei der Steinzertrümmerung und Anordnung zur Durchführung des Verfahrens.

(30) Priorität: 10.10.85 DE 3536144

(43) Veröffentlichungstag der Anmeldung:
13.05.87 Patentblatt 87/20

(45) Bekanntmachung des Hinweises auf die Patenterteilung: 09.08.89 Patentblatt 89/32

(84) Benannte Vertragsstaaten:
DE FR GB

(56) Entgegenhaltungen:
EP–A– 0 148 653
EP–A– 0 194 897
DE–A– 2 722 252
GB–A–  998 173

(73) Patentinhaber: Philips Patentverwaltung GmbH
Wendenstrasse 35 Postfach 10 51 49
D-2000 Hamburg 1 (DE)
DE
N.V. Philips' Gloeilampenfabrieken
Groenewoudseweg 1
NL-5621 BA Eindhoven (NL)
FR GB

(72) Erfinder: Zimmer, Hildebrand
Hinterm Vogelherd 65
D-2070 Ahrensburg (DE)

(74) Vertreter: Hartmann, Heinrich, Dipl.-Ing. et al
Philips Patentverwaltung GmbH Wendenstrasse 35
Postfach 10 51 49
D-2000 Hamburg 1 (DE)

EP 0 221 592 B1

## Beschreibung

Die Erfindung betrifft ein Verfahren zur Ultraschallkontrolle bei der Zertrümmerung von Steinen in Körpern von Lebewesen, wobei zus Ultraschallbeobachtung periodisch eine Ultraschallwandleranordnung in einer Sendephase elektrische Energie in akustische Energie und in einer Empfangsphase akustische Echosignale in elektrische Signale umsetzt sowie eine Anordnung zur Durchführung eines solchen Verfahrens. Eine solche Anordnung ist aus EP-A1-0 148 653 bekannt.

Aus « Röntgenstrahlen » Heft 49, 1983, Seiten 4 bis 9 ist eine Anordnung bekannt, in welcher die Kontrolle der Steinzertrümmerung sowie die vorangehende Ortung des Steines mit Hilfe einer Anordnung aus zwei Röntgenstrahlern und zwei Bildverstärkern erfolgt, jedoch ist auch die Ultraschallortung erwähnt worden, wobei jedoch angegeben ist, daß mit Hilfe von Ultraschall eine Kontrolle des Behandlungserfolges nicht möglich ist, weil im Ultraschallbild eine Differenzierung zwischen dem zertrümmerten Stein und einem nicht zertrümmerten Stein nicht möglich ist. Diese Feststellungen beziehen sich zwar nur auf Nierensteine, doch gelten sie in gleicher Weise auch für Gallensteine. Ein Gallenstein liefert im Ultraschallbild ein Echo, das sich kaum von dem Echo von Gesteinstrümmern am Boden der Gallenblase unterscheidet.

Es ist Aufgabe der vorliegenden Erfindung ein Verfahren der eingangs genannten Art so auszugestalten, daß eine Differenzierung zwischen einem kompakten Gallenstein und Gallengesteinsträmmern in der Gallenblase mit Hilfe von Ultraschall möglich wird.

Diese Aufgabe wird erfindungsgemäß dadurch gelöst, daß vor der Ultraschallbeobachtung die Ultraschallwandleranordnung während eines im Vergleich zur Sendephase großen Zeitraums mit einer im Vergleich zur elektrischen Leistung während der Sendephase hohen elektrischen Leistung beaufschlagt wird, wodurch der Stein bzw. Steintrümmer aufgewirbelt werden.

Wenn die Ultraschallwandleranordnung während eines relativ langen Zeitraums mit einer relativ hohen elektrischen Leistung beaufschlagt wird, erzeugt er aufgrund des Schallstrahlungsdruckes einen mechanischen Impuls, der ausreicht, um den Gallenstein oder die Gallensteinsträmmer in der Gallenblase aufzuwirbeln. Nach diesem Aufwirbeln können Gallensteine und Gallensteinsträmmer deutlich voneinander unterschieden werden, weil sie aufgrund ihres spezifischen Gewichtes und der Viskosität der Gallenflüssigkeit abhängig von ihrer Größe mehr oder weniger schnell auf den Fundus der Gallenblase absinken. Bei der Ultraschallbeobachtung dieses Vorganges kann sowohl aus der Größe der Echos der Auslöschungsschatten als auch der Zeit zum Absetzen auf die Größe der in der Gallenblase enthaltenen Steine bzw. Steintrümmer geschlossen werden. Um einen Gallenstein aufwirbeln zu können, sollte während einer Zeit zwischen 5 ms und 250 ms eine akustische Leistung von wenigstens 30 W (bei einem Steingewicht von 1 g) auf ihn einwirken.

Bei der Erfindung hat die Ultraschallwandleranordnung eine dreifache Funktion :

Vor der eigentlichen Ultraschalluntersuchung wirbelt sie die Steine bzw. die Gesteinsträmmer hoch, während der Ultraschalluntersuchung liefert sie die Sendeimpulse und setzt die akustischen Echosignale in elektrische Signale um, die zu einer bildlichen Darstellung der aufgewirbelten Gesteinsträmmer dienen.

Die Anordnung zur Durchführung des Verfahrens, die von einer mit einer Flüssigkeit gefüllten Wanne zur Aufnahme des Körpers, einer Einrichtung zum Erzeugen von Stoßwellen, einer Einrichtung zur Fokussierung der Stoßwellenenergie auf einen Punkt in der Wanne und mit einer Ultraschallwandleranordnung die in einer ersten Betriebsart als Sende- und Empfangswandler eines Ultraschalluntersuchungsgerätes betreibbar ist ausgeht, zeichnet sich dadurch aus, daß eine Umschaltvorrichtung vorgesehen ist, durch die die Ultraschallwandleranordnung in einer zweiten Betriebsart vor der Ultrahallbeobachtung mit einer im Vergleich zur ersten Betriebsart großen elektrischen Leistung während eines relativ großen Zeitraums beaufschlagt ist, wodurch der Stein bzw. Steintrümmer aufgewirbelt werden.

Eine Weiterbildung, die davon ausgeht, daß die Einrichtung zur Fokussierung der Stoßwellen ein Rotationsellipsoid mit zwei Brennpunkten umfaßt, wobei die Stoßwelle in dem einen Brennpunkt erzeugt und auf den anderen Brennpunkt fokussiert wird, sieht vor, daß die Ultraschallwandleranordnung auf der Verbindungslinie der beiden Brennpunkte angeordnet ist. Diese Weiterbildung ist insofern günstig, als die Ultraschallenergie aus der gleichen Richtung in den Körper eindringt wie die Energie der Stoßwelle.

In weiterer Ausgestaltung ist vorgesehen, daß ein Antrieb zur Verschiebung der Ultraschallwandleranordnung senkrecht zur Verbindungslinie der Brennpunkte angeordnet ist. Hierdurch kann gegebenenfalls vermieden werden, daß die Ultraschallwandleranordnung einen größeren Teil der Ellipsoidapertur abdeckt und damit die Stoßwellen auf dem Wege zum Fokussierungspunkt dämpft.

Nach einer weiteren Ausgestaltung der Erfindung ist vorgesehen, daß die Ultraschallwandleranordnung einen einzigen Schallschwinger umfaßt. Grundsätzlich könnte die Ultraschallwandleranordnung auch eine Vielzahl von Ultraschallschwingern (array) umfassen, die die Signale mit geeigneter elektronisch steuerbarer Phasenverschiebung aussenden bzw. verarbeiten. Da im vorliegenden Fall sich der Gallenstein bzw. die Gallensteinsträmmer im definierten Abstand von der Ultraschallwandleranordnung befinden, ist eine elektronische Fokussierung nicht erforderlich. Es kann vielmehr mit einer festen durch den Aufbau des Ultraschallschwingers vorgegebenen Fokuseinstellung gearbeitet werden. Ein einzelner Ultraschallschwinger kann relativ einfach so ausgelegt werden, daß er die erforderlichen Schallintensitäten erzeugen kann.

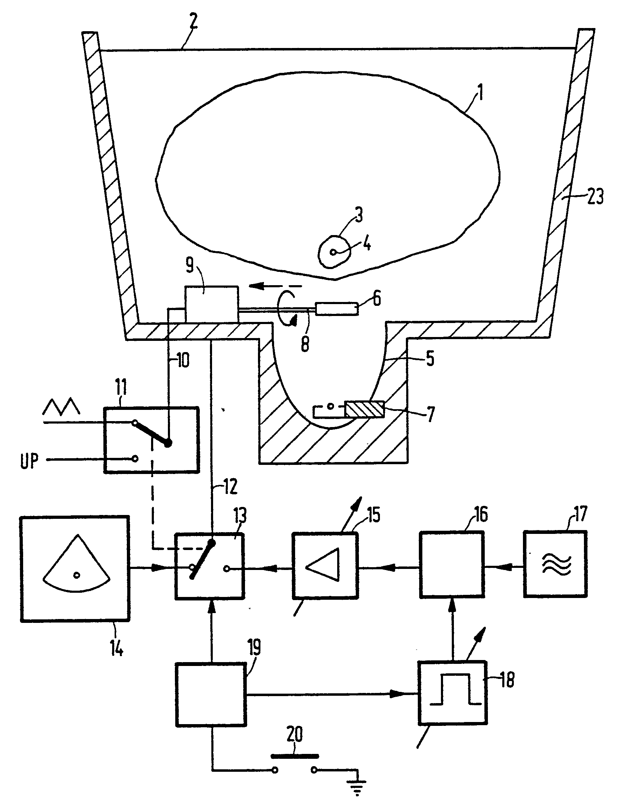

Die Erfindung wird nachstehend anhand eines in der Zeichnung dargestellten Ausführungsbeispiels näher erläutert.

Die Zeichnung zeigt einen menschlichen Körper 1 im Querschnitt, der sich in einer mit einer Flüssigkeit 2, z. B. Wasser, gefüllten Wanne 23 befindet. Die Gallenblase ist mit 3 bezeichnet und ein darin befindlicher Stein mit 4. In der Mitte des Bodens der Wanne befindet sich eine rotationsellipsoidförmige Vertiefung 5, in deren einen Brennpunkt eine Funkenstrecke 7 angeordnet ist, die Stoßwellen erzeugen kann, die im anderen Brennpunkt des Rotationsellipsoids fokussiert werden. Zwischen den beiden Brennpunkten, d. h. auf der Symmetrieachse des Rotationsellipsoids, befindet sich ein Ultraschallschwinger 6 mit einer Resonanzfrequenz von z. B. 3 MHz. Er ist auf den Brennpunkt fokussiert in dem die durch die Funkenstrecke 7 erzeugten Schockwellen zusammenlaufen. In diesem Brennpunkt befindet sich bei der Erzeugung der Schockwellen bzw. der Ultraschalluntersuchung der Gallenstein 4 bzw. der tiefste Punkt der Gallenblase 3.

Der Ultraschallschwinger 6 ist an einer horizontal, d. h. senkrecht zur Verbindungslinie der Brennpunkte, verlaufenden Welle 8 befestigt. Auf diese Welle wirkt ein Antrieb 9 ein, dem über eine Leitung 10 und eine Umschaltvorrichtung 11 entweder ein dreieckförmiges Steuersignal oder eine konstante Spannung UP — gegebenenfalls auch Massepotential — zugeführt wird. Wenn der Antrieb 9 mit dem dreieckförmigen Steuersignal beaufschlagt wird, führt der Ultraschallschwinger eine mit einer Periode von z. B. 100 ms hin und hergehende Winkelbewegung aus, die einen bestimmten zur Zeichenebene senkrechten und durch den zweiten Brennpunkt des Ellipsoids 5 verlaufenden Sektor bestreicht, so daß in dieser Richtung Ultraschall emittiert oder empfangen werden kann.

Der Ultraschallschwinger 6 ist über eine Leitung 12 mit einem weiteren Umschalter 13 verbunden, an den einerseits die Komponenten eines konventionellen Ultraschalluntersuchungsgerätes 14 (Sender, Empfänger, Scanconverter, Monitor) für die Realzeit-Sektorbilddarstellung enthalten ist und andererseits der Ausgang eines Leistungsverstärkers 15 mit regelbarer Verstärkung. Der Eingang des Verstärkers 15 ist über einen Modulator 16 mit einem Hochfrequenzerzeuger 17 verbunden. Der Hochfrequenzerzeuger 17 liefert eine Spannung mit einer Frequenz von 3 MHz, d. h. mit der Resonanzfrequenz des Ultraschallschwingers 6. Der Modulator 16 wird von einem Impulserzeuger 18 gesteuert, der Impulse mit einer zwischen 5 ms und 250 ms einstellbaren Impulsdauer liefert. Die Impulsdauer soll nicht zu kurz oder zu lang sein. Bei sehr kurzen Impulsen ist eine hohe Leistung erforderlich und bei sehr langen, relativ leistungsschwachen Impulsen geraten die Gallensteine nur ins Schweben und werden nicht hochgewirbelt. Nur wenn ein solcher Impuls geliefert wird, erzeugt der Modulator 16 ein Ausgangssignal mit der Frequenz der vom Generator 17 erzeugten Schwingung ; liefert der Impulserzeuger keinen Impuls, ist das Ausgangssignal des Modulators 16 Null. Eine Steuereinrichtung 19 schaltet bei Betätigung eines Schalters 20 durch den Benutzer die Umschaltvorrichtungen 11, 13 kurzzeitig aus der dargestellten Schaltstellung in die nicht dargestellte Schaltstellung und aktiviert den Impulserzeuger 18, z. B. einen monostabilen Multivibrator, so daß dieser einen einzigen Impuls erzeugt.

Die Anordnung wird auf folgende Weise betrieben :

Vor der Stoßwellenerzeugung wird zunächst eine Ultraschallbeobachtung durchgeführt, bei der der Gallenstein lokalisiert wird. Der Körper 1 wird dann mittels einer nicht näher dargestellten Hebevorrichtung in der Wanne 23 so positioniert, daß der Gallenstein 4 am Fundus der Gallenblase sich am Ort des zweiten Brennflecks befindet, was im Ultraschallbild daran zu erkennen ist, daß das Bild des Gallensteins sich an einer bestimmten Stelle des Bildschirms der Wiedergabeeinrichtung des Untersuchungsgerätes 14 befindet. Bei der Ultraschalluntersuchung befinden sich die Umschaltvorrichtungen 11 und 13 in der in der Zeichnung dargestellten Schaltstellung. Dem Motorantrieb 9 wird dann das dreieckförmige Signal zugeführt, so daß er eine Periodische hin und hergehende Winkelbewegung ausführt, wobei während jeder Periode von etwa 100 ms der Ultraschallschwinger 6 mit konstanter Impulswiederholungsfrequenz 256 mal in der Sendephase kurz (z. B. 1 μs) angeregt wird, während in der Zeit zwischen zwei Sendeimpulsen die Ultraschallechos in elektrische Signale umgesetzt werden, die in dem Scanconverter digital gespeichert und auf einem Monitor in ein sichtbares Ultraschallsektorbild umgesetzt werden.

Wenn auf diese Weise der Gallenstein und der Ultraschallbeobachtung in den zweiten Brennpunkt des Rotationsellipsoids gerückt ist, wird die Funkenstrecke ein oder mehrmals gezündet. Die dabei gegebenenfalls erzeugten Steintrümmer setzen sich im Fundus der Gallenblase 3 ab, d. h. an der gleichen Stelle, an der sich vorher der kompakte Gallenstein befand.

Um die Zertrümmerung des Steins kontrollieren zu können, wird der Tastschalter 20 betätigt, wodurch einerseits die Schaltung 18 zur Impulserzeugung aktiviert wird und andererseits die Umschaltvorrichtungen 11, 13 für einen Zeitraum, der kurz nach dem Ende des Impulses endet, in die nicht dargestellte Schaltstellung umgeschaltet werden. In dieser Stellung der Schaltvorrichtung 11 nimmt der Ultraschallschwinger eine definierte Winkelstallung im Raum ein, und zwar die, in welcher ein von ihm erzeugter Ultraschallstrahl den Gallenstein 4 durchsetzt. Die dem Ultraschallschwinger 6 in dieser Stellung der Umschaltvorrichtung 13 zugeführte elektrische Leistung ist so groß, daß die vom Ultraschallschwinger 6 erzeugte akustische Energie ausreicht, um einen Gallenstein in der Gallenblase um einige cm anzuheben. Je nach dem — durch den Auftrieb in der Gallenflüssigkeit wesentlich herabgesetzten — Gewicht des Gallensteines ist sie mehr oder weniger groß; sie beträgt bei einem Gewicht von 50 mg — was etwa einem Gallenstein von 1 cm$^3$ entspricht — rund 200 Ws (für 5 cm Anhebung). Dadurch wird der Gallenstein bzw. die Gallensteintrümmer in der Gallenblase hochgewirbelt und ihr Absinken kann in der unmittelbar an das Hochwirbeln anschließenden Ultraschalluntersuchungsphase beobachtet und ausgewertet werden.

Die Öffnung des Rotationsellipsoids ist wesentlich größer als der Durchmesser des Ultraschallschwingers. Dieser schattet daher den Gallenstein nur unwesentlich gegenüber der von der Funkenstrecke 7 ausgehenden Stoßwelle ab- jedenfalls dann, wenn er sich nicht zu dicht beim zweiten Brennpunkt befindet. Um eine etwaige Abschattung oder auch eine Beschädigung des Ultraschallschwingers durch die Stoßwelle ganz auszuschließen, kann es von Nutzen sein, den Ultraschallschwinger 6 mittels eines nicht näher dargestellten Antriebs vor der Zündung der Funkenstrecke 7 seitlich so weit zu verschieben, daß er die Öffnung des Rotationsellipsoids nicht mehr bedeckt.

Die vorstehende Anordnung verwendet eine Wanne 23, in der sich der Körper 1, eine Koppelflüssigkeit 2 — Wasser — usw. befinden. Es ist aber auch möglich, die Koppelflüssigkeit in einen flexiblen Sack oder Schlauch aufzunehmen und die Funkenstrecke darüber an den Körper anzukoppeln. Der Ultraschallschwinger muß dann in dem Schlauch bzw. Sack sein.

**Patentansprüche**

1. Verfahren zur Ultraschallkontrolle bei der Zertrümmerung mittels Stoßwellen von Steinen in Körpern von Lebewesen insbesondere zur Differenzierung zwischen zertrümmerten und nicht zertrümmerten Steiner wobei zur Ultraschallbeobachtung periodisch eine Ultraschallwandleranordnung in einer Sendephase elektrische Energie in akustische Energie und in einer Empfangsphase akustische Echosignale in elektrische Signale zwecks Bilderstellung umsetzt, dadurch gekennzeichnet, daß vor der Ultraschallbeobachtung die Ultraschallwandleranordnung (6) während eines im Vergleich zur Sendephase großen Zeitraums mit einer im Vergleich zur elektrischen Leistung während der Sendephase hohen elektrischen Leistung beaufschlagt wird, wodurch der Stein bzw. Steintrümmer aufgewirbelt werden.

2. Anordnung zur Durchführung des Verfahrens nach Anspruch 1 mit einer Einrichtung (7) zur Erzeugung von Stoßwellen, die über ein Koppelmedium (2) auf den Körper (1) einwirken, einer Einrichtung (5) zur Fokussierung der Stoßwellen auf einen Punkt in dem Körper und mit einer Ultraschallwandleranordnung (6) zur Ultraschallbeobachtung, die in einer ersten Betriebsart als Sende- und Empfangswandler eines Ultraschalluntersuchungsgerätes (14) betriebbar ist, dadurch gekennzeichnet, daß eine Umschaltvorrichtung (11, 13) vorgesehen ist, durch die die Ultraschallwandleranordnung in einer zweiten Betriebsart vor der Ultrachallbeobachtung mit einer im Vergleich zur ersten Betriebsart großen elektrischen Leistung während eines relativ großen Zeitraums beaufschlagbar ist wodurch der Stein bzw. Steintrümmer aufgewirbelt werden.

3. Anordnung nach Anspruch 2, wobei die Einrichtung zur Fokussierung der Stoßwellenenergie ein Rotationsellipsoid (5) mit zwei Brennpunkten umfaßt, und wobei die Stoßwelle in dem einen Brennpunkt erzeugt und auf den anderen Brennpunkt fokussiert wird, dadurch gekennzeichnet, daß die Ultraschallwandleranordnung (6) auf der Verbindungslinie der beiden Brennpunkte angeordnet ist.

4. Anordnung nach Anspruch 3, gekennzeichnet durch einen Antrieb zur Verschiebung der Ultraschallwandleranordnung senkrecht zur Verbindungslinie der Brennpunkte.

5. Anordnung nach Anspruch 2, dadurch gekennzeichnet, daß die Ultraschallwandleranordnung einen einzigen Ultraschallschwinger (6) umfaßt.

**Claims**

1. A method for the ultrasonic checking of the smashing of calculi, by means of shock waves, in bodies of living beings, notably for discrimination between smashed and non-smashed calculi, where for ultra-sonic observation an ultrasound transducer device periodically converts electric energy into acoustic energy during a transmission phase, and converts acoustic echo signals into electric signals for imaging during a receiving phase, characterized in that prior to the ultrasound observation, an electric power which is high in comparison with the electric power applied during the transmission phase is applied to the ultrasound transducer device (6) during a period of time which is long in comparison with the transmission phase, thus stirring up the calculus or calculus fragments.

2. An apparatus for performing the method claimed in Claim 1, including a device (7) for generating shock waves which act on the body (1) via a coupling medium (2), a device (5) for focussing the shock waves onto one point inside the body, and an ultrasound transducer device (6) for ultrasound observation which can operate as a transmitter/receiver transducer of an ultrasonic examination apparatus (14) in a first mode of operation, characterized in that the apparatus comprises a switching device (11, 13) which enables an electric power which is high in comparison with that applied during the first mode of operation to be applied to the ultrasound transducer device in a second mode of operation for a comparatively long period of time, thus stirring up the calculus or calculus fragments.

3. An apparatus as claimed in Claim 2 in which the device for focussing the shock wave energy comprises an ellipsoid of revolution (5) having two focal points and in which the shock wave is generated in one focal point and is focussed onto the other focal point, characterized in that the ultrasound transducer device (6) is arranged on the connecting line between the two focal points.

4. An apparatus as claimed in Claim 3, characterized in that it comprises a drive for displacing the ultrasound transducer device perpendicularly to the connecting line between the focal points.

5. An apparatus as claimed in Claim 2, characterized in that the ultrasound transducer device comprises a single ultrasound transducer (6).

**Revendications**

1. Procédé de contrôle par ultrasons lors de la destruction de calculs dans des corps d'êtres vivants au moyen d'ondes de choc, notamment pour la différenciation de calculs détruits et non détruits, suivant lequel, pour l'observation par ultrasons, un dispositif transducteur ultrasonore convertit de l'énergie électrique en énergie acoustique dans une phase d'émission et des signaux d'écho acoustiques en signaux électriques dans une phase de réception, caractérisé en ce qu'avant l'observation par ultrasons, durant un intervalle de temps long par rapport à la phase d'émission, est fournie au dispositif transducteur ultrasonore (6) une puissance électrique élevée par rapport à la puissance électrique fournie au cours de la phase d'émission, de sorte que le calcul ou les fragments de calcul sont soulevés en tourbillons.

2. Dispositif pour la mise en oeuvre du procédé selon la revendication 1, muni d'un dispositif (7) pour engendrer des ondes de choc agissant sur le corps (1) à travers un milieu de couplage (2), d'un dispositif (5) pour focaliser les ondes de choc sur un point dans le corps et d'un dispositif transducteur ultrasonore (6) pour l'observation par ultrasons qui, dans un premier mode de fonctionnement, fait fonction de transducteur d'émission et de réception d'un appareil d'examen par ultrasons (14), caractérisé en ce qu'il est prévu un dispositif de commutation (11, 13), au moyen duquel, dans un second mode de fonctionnement, avant l'observation par ultrasons, le dispositif transducteur ultrasonore reçoit durant un intervalle de temps relativement long une puissance électrique élevée par rapport au premier mode de fonctionnement, de sorte que le calcul ou les fragments de calcul sont soulevés en tourbillons.

3. Dispositif selon la revendication 2, dans lequel le dispositif de focalisation de l'énergie d'onde de choc comporte un ellipsoïde de révolution (5) ayant deux foyers et dans lequel l'onde de choc est engendrée dans l'un des foyers et est focalisée sur l'autre foyer, caractérisé en ce que le dispositif transducteur ultrasonore (6) est disposé sur la ligne de jonction des deux foyers.

4. Dispositif selon la revendication 3, caractérisé par un dispositif d'entraînement pour déplacer le dispositif transducteur ultrasonore perpendiculairement à la ligne de jonction des foyers.

5. Dispositif selon la revendication 2, caractérisé en ce que le dispositif transducteur ultrasonore comporte un seul élément transducteur ultrasonore (6).